# EUROPEAN PATENT APPLICATION

(11) **EP 4 814 106 A1**
(43) Date of publication of application: **30.09.2026**
(21) Application number: 24893624.7
(22) Date of filing: 23.11.2024
(51) Int. Cl.: C12N 1/21, C12P 5/00, C12N 15/70, C12P 7/42, C12R 1/19

(54) **CO-CULTURE SYSTEM AND METHOD FOR SYNTHESIZING TARGET COMPOUND**

(30) Priority: 24.11.2023 CN 202311583460
(71) Applicant: Oxford University (Suzhou) Science & Technology Co., Ltd., Suzhou, Jiangsu 215123 (CN)
(72) Inventor: WONG, Luet, Oxfordshire (GB); PANDREKA, Avinash, Andhra Pradesh (IN); CAO, Yang, Suzhou, Jiangsu 215123 (CN)
(74) Representative: Dr. Solf & Zapf Patent- und Rechtsanwalts PartG mbB
(86) International application number: PCT/CN2024/134030
(87) International publication number: WO 2025/108471

(57) **Abstract**

The present invention discloses a co-cultivation system for synthesizing a target compound, the co-cultivation system comprising at least one feeding strain and at least one production strain, wherein the feeding strain provides a precursor compound, and the production strain synthesizes the target compound using the precursor compound, the co-cultivation system further comprising an antibiotic, and wherein one of the production strain and the feeding strain is resistant to the antibiotic, and the other is not resistant to the antibiotic. The present invention also discloses a method for synthesizing a target compound by means of the co-cultivation system.

## Description

### Technical field

The present invention relates to a co-cultivation system and method for the synthesis of a target compound, and particularly to the control of strain dynamics in a co-cultivation system for synthesizing the target compound.

### Background

Terpenoids are widely used in different industrial applications such as flavors, fragrances, biofuels, pharmaceuticals, rubber, and pesticides. These compounds are produced by plants, but often in low levels that are not enough to meet industrial needs.

Terpenoids refer to compounds derived from mevalonic acid or 1-deoxy-D-xylulose 5-phosphate and having molecular skeletons based on isoprene units (C5 units) as the basic structural units, and oxygenated derivatives of such compounds. These oxygenated derivatives may include alcohols, aldehydes, ketones, carboxylic acids, esters, and the like. Most metabolites produced during terpenoid biosynthesis are structurally complex, and their chemical production generally involves multiple steps and complex chemical reactions, thereby resulting in low yields, incorrect stereochemistry, and high costs.

In one process for synthesizing terpenoids, two strains, such as a feeding strain and a production strain, are grown in medium according to the isoprene unit biosynthetic pathway to obtain terpenoids. However, the populations of the strains may change during the growth phase, leading to variations in total terpenoids yield. Under different conditions and metabolic loads, the faster-growing strain will dominate the cultivation. This makes it currently impossible to control the ratio of the feeding strain to the production strain within a desired range throughout the cultivation process.

In the prior art, several methods exist for regulating the growth rate of strains (such as mutant strains) in co-cultivation systems (Aulakh, Simran Kaur et al., Nature Chemical Biology (2023): 1-11. https://doi.org/10.1038/s41589-023-01341-2), toxin-antitoxins (US10188114B2)). In addition, some technologies use quorum sensing (Scott et al., Nature Microbiology, 2017, 2, 17083; DOI: 10.1038/nmicrobiol.2017.83) or optogenetics (Lalwani, Makoto A., et al., ACS Synthetic Biology 10.8 (2021): 2015-2029. doi.org/10.1021/acssynbio.1c00182) to control strain growth. These methods control strain growth by adjusting antibiotic resistance or the expression of toxic proteins (Gutiérrez Mena, Joaquín, Sant Kumar, and Mustafa Khammash, Nature Communications 13.1 (2022): 4808. https://doi.org/10.1038/s41467-022-32392-z; Yurtsev, Eugene Anatoly, Arolyn Conwill, and Jeff Gore. Proceedings of the National Academy of Sciences 113.22 (2016): 6236-6241. www.pnas.org/cgi/doi/10.1073/pnas.1523317113; Liu, Feng, et al., ACS Synthetic Biology 8.8 (2019): 1713-1722. DOI: 10.1021/acssynbio.9b00110; Blanchard, Andrew E., Chen Liao, and Ting Lu, Cellular and Molecular Bioengineering 9 (2016): 443-454. https://doi.org/10.1007/s12195-016-0447-6; Fedorec, Alex JH, et al. Nature Communications 12.1 (2021): 1977. https://doi.org/10.1038/s41467-021-22240-x). Most of these systems require knocking out essential genes or the expression of enzymes to obtain response elements and growth regulators; however, expression of control systems together with heterologous pathways places additional stress on the microorganisms.

### SUMMARY OF THE INVENTION

To overcome deficiencies in the prior art, the present invention provides a co-cultivation system for synthesizing a target compound, comprising at least one feeding strain and at least one production strain, wherein the feeding strain provides a precursor compound, and the production strain synthesizes the target compound using the precursor compound, the co-cultivation system further comprising an antibiotic, and wherein one of the production strain and the feeding strain is resistant to the antibiotic, and the other is not resistant to the antibiotic.

By means of the co-cultivation system according to the present invention, the ratio of the production strain to the feeding strain can be dynamically adjusted in a simple and accurate manner, so that the growth dynamics of the microorganisms can be controlled while the stress on the microorganisms expressing heterologous enzymes is reduced.

According to some advantageous embodiments of the present invention, in the co-cultivation system, of the production strain and the feeding strain, the faster-growing one is sensitive to the antibiotic, and the slower-growing one is resistant thereto. In some preferred embodiments, the faster-growing strain is the feeding strain, and the slower-growing strain is the production strain.

According to some embodiments of the present invention, the feeding strain is not resistant to the antibiotic, and the production strain is resistant to the antibiotic.

According to some embodiments of the present invention, the target compound is a terpenoid.

According to some embodiments of the present invention, the precursor compound is mevalonic acid.

According to some embodiments of the present invention, the feeding strain is used to provide mevalonic acid, and the production strain synthesizes terpenoid using the mevalonic acid.

According to some embodiments of the present invention, the feeding strain contains a gene for synthesizing mevalonic acid, which is overexpressed and does not contain a gene for synthesizing farnesyl pyrophosphate.

According to some embodiments of the present invention, the production strain contains a first plasmid and a second plasmid, wherein the first plasmid contains a gene for synthesizing farnesyl pyrophosphate and does not contain a gene for synthesizing mevalonic acid, and the second plasmid contains a gene of the terpenoid synthase.

According to some embodiments of the present invention, the target compound includes at least one of a monoterpenoid, a sesquiterpenoid, and a diterpenoid.

According to some embodiments of the present invention, the monoterpenoid includes at least one of linalool, geraniol, and 2,6-dimethyloctane; the sesquiterpenoid includes at least one of bisabolene, pentalenene, isohimachalene, bulnesene, valencene, β-patchoulene, aristolochene, guaiene, cedrene, oxidized bulnesene, and oxidation products of valencene; and/or the diterpenoid is taxadiene.

According to some embodiments of the present invention, the terpenoid includes at least one of linalool, geraniol, 2,6-dimethyloctane, bisabolene, pentalenene, isohimachalene, taxadiene, bulnesene, valencene, β-patchoulene, and aristolochene.

According to some embodiments of the present invention, the terpenoid includes at least one of an oxidation product of bulnesene or valencene.

According to some embodiments of the present invention, some antibiotics act extracellularly by inhibiting cell wall synthesis, while others act intracellularly by inhibiting gene translation. In some embodiments, the antibiotic in the co-cultivation system includes at least one of chloramphenicol, streptomycin, kanamycin, and tetracycline.

According to some preferred embodiments of the present invention, the antibiotic is an intracellular antibiotic, preferably streptomycin.

According to some embodiments of the present invention, the concentration of the antibiotic is set such that cells of the strain that is not resistant to the antibiotic, i.e., the antibiotic-sensitive strain, are not killed but only have their growth rate restricted, while cells of the strain that is resistant to the antibiotic are not affected by the antibiotic.

According to some embodiments of the present invention, the initial concentration of the antibiotic is set such that the growth rate of feeding cells that are not resistant to the antibiotic is inhibited by the antibiotic.

According to some embodiments of the present invention, since the strain in the co-cultivation system that is resistant to the antibiotic will degrade the antibiotic, the strain that is not resistant to the antibiotic will gradually become unaffected by the antibiotic. For example, the production strain may gradually degrade the antibiotic during incubation, so as to weaken the effect of the antibiotic on the feeding strain. In this case, the growth rate of the feeding strain depends on the initial concentration of the antibiotic and the degradation rate of the antibiotic, and the degradation rate of the antibiotic depends on the growth rate of the production strain. Thus, the growth rate of the production strain indirectly controls the growth rate of the feeding strain.

According to some embodiments of the present invention, based on the co-cultivation system, the concentration of the antibiotic is at least 5 µg/mL, and/or at most 20 µg/mL.

According to some embodiments of the present invention, based on the co-cultivation system, the concentration of the antibiotic is from 5 µg/mL to 20 µg/mL, such as 5 µg/mL, 6 µg/mL, 7 µg/mL, 8 µg/mL, 9 µg/mL, 10 µg/mL, 11 µg/mL, 12 µg/mL, 13 µg/mL, 14 µg/mL, 15 µg/mL, 16 µg/mL, 17 µg/mL, 18 µg/mL, 19 µg/mL, 20 µg/mL, or any range constituted thereby.

According to some embodiments of the present invention, based on the co-cultivation system, the antibiotic concentration is from 5 µg/mL to 12.5 µg/mL.

According to some embodiments of the present invention, based on the co-cultivation system, the antibiotic concentration is from 5 µg/mL to 10 µg/mL.

According to some embodiments of the present invention, the production strain is transformed with the pMBIS and pTRC-BS plasmids. The production strain is thereby capable of producing bulnesene.

According to some embodiments of the present invention, the production strain is transformed with at least one of the plasmids pCDF-FIAMAV and pCDF-FIAMAV-V1. The production strain is thereby resistant to the antibiotic in the co-cultivation system, and oxidizes bulnesene into bulnesene-15-ol and bulnesene-15-al.

According to some embodiments of the present invention, an antibiotic resistance plasmid has been removed from the feeding strain, so that the feeding strain has higher sensitivity to the antibiotic, whereby the initial concentration of the antibiotic in the co-cultivation system can be reduced, and the biological stress imposed on the two strains can thereby be reduced.

Another object of the present invention is to provide a method for synthesizing a target compound, which synthesizes the target compound by means of the co-cultivation system according to the present invention.

According to some embodiments of the present invention, the concentration of the antibiotic is set such that the ratio of the feeding strain to the production strain is adjusted in accordance with the concentration of the antibiotic. For example, an appropriate initial concentration of the antibiotic is set in order to obtain a desired ratio of the number of feeding strain cells to the number of production strain cells during incubation.

According to some embodiments of the present invention, when the inoculation ratio of the feeding strain to the production strain exceeds 25%:75%, the concentration of the antibiotic may also be gradually increased, for example, the concentration of the antibiotic may be increased to 5 µg/mL to 20 µg/mL or any value therebetween. The total terpenoid yield level can be used to determine the antibiotic concentration required for a particular ratio of the feeding strain to the production strain. The growth of the feeding strain can thereby be appropriately controlled.

According to some embodiments of the present invention, the feeding strain and/or the production strain are selected from one or more of bacteria, fungi, or yeasts. According to some specific embodiments of the present invention, the feeding strain and/or the production strain is *E. coli*.

According to some embodiments of the present invention, the ratio of the feeding strain to the production strain is (10-90): (90-10). According to some preferred embodiments of the present invention, the ratio of the feeding strain to the production strain is (12.5-75): (87.5-25). In the present invention, the range of the ratio of the production strain to the feeding strain can also be controlled by changing the inoculation ratio, thereby further improving the yield of terpenoid.

According to some embodiments of the present invention, based on the total inoculation volume of the feeding strain and the production strain, the content of the feeding strain is from 10% to 90%. According to a preferred embodiment of the present invention, based on the inoculation volume of the feeding strain and the production strain, the content of the feeding strain is from 12.5% to 75%, for example, 12.5%, 25%, 37.5%, 50%, 62.5%, 75%, and any value therebetween.

The co-cultivation system according to the present invention can improve the total terpenoid output. When carried out in shake flasks, the co-cultivation system according to the present invention can yield approximately 500 mg/L to 2500 mg/L of terpenoid, and the yield will increase significantly when the co-cultivation system is transferred to a fermenter.

By means of the co-cultivation system according to the present invention, any terpenoid can be produced by expressing the corresponding terpene synthase, including but not limited to monoterpenoids for biofuel production (linalool, geraniol, and 2,6-dimethyloctane) and other sesquiterpenoids (bisabolene, pentalenene, and isohimachalene), and diterpenoids (taxadiene) for pharmaceutical and other applications.

According to some embodiments of the present invention, the feeding strain can be further modified to express hydrolase enzymes to degrade polymers such as cellulose, lignin, hemicellulose, and the like; in some embodiments, multiple feeding strains can be developed and controlled by an antibiotic system that produces terpenoid by utilizing or degrading complex biological materials; in some embodiments, the antibiotic-controlled co-cultivation principle can be combined with other pathways to enhance microbial community interactions, fluxes, and product formation; in some embodiments, different microorganisms such as yeasts, fungi, and bacteria can be used to create multi-strain co-cultivations, and their numbers can be controlled by antibiotics during both the growth phase and the expression phase.

### Description of drawings

Figure 1 schematically shows the yields of single-strain and co-cultivation systems for the biosynthesis of bulnesene and its oxidation products using pCDF-FIAMAV.
Figure 2 schematically shows the yields of single-strain and co-cultivation systems for the biosynthesis of bulnesene and its oxidation products using pCDF-FIAMAV-V1.
Figure 3 schematically shows the yield of the co-cultivation system of Example 1.
Figure 4 schematically shows the yield of the co-cultivation system of Example 2.
Figure 5 schematically shows the yield of the co-cultivation system of Example 3.
Figure 6 schematically shows the yield of the co-cultivation system of Example 4.

### EMBODIMENT

The present application will be further described below in conjunction with specific embodiments. It should be understood that these specific embodiments are only used to illustrate the present application and not to limit the scope of the present application.

For the sake of brevity, only some numerical ranges are specifically disclosed herein. However, any lower limit can be combined with any upper limit to form an unspecified range; and any lower limit can be combined with any other lower limit to form an unspecified range, and likewise any upper limit can be combined with any other upper limit to form an unspecified range. Furthermore, each individually disclosed point or single value may itself serve as a lower or upper limit in combination with any other point or single value or with other lower or upper limits to form a range that is not expressly recited.

Unless otherwise specified, terms used in this application have their commonly known meanings as commonly understood by those skilled in the art. Unless otherwise specified, the numerical values of the parameters mentioned in this application can be measured by various measurement methods commonly used in the art (for example, can be tested according to the methods given in the examples of this application).

A list of items to which the terms "at least one of," or other similar terms are linked can mean any combination of the listed items. For example, if items A and B are listed, the phrase "at least one of A and B" means A only; B only; or A and B. In another example, if items A, B, and C are listed, the phrase "at least one of A, B, and C" means A only; or B only; C only; A and B (excluding C); A and C (excluding B); B and C (excluding A); or all of A, B, and C. Item A may contain a single component or multiple components. Item B may contain a single component or multiple components. Item C may contain a single component or multiple components.

The pMVA, pMBIS, and pMeVT plasmids are resistant to chloramphenicol and were generated by methods similar to those disclosed in Chinese patent application No. 2022105905014 (international application No. PCT/CN2023/096509).

In the examples of the present invention, the codon-optimized bulnesene synthase gene (NCBI - KF800046) was cloned into pTRC-HisA to generate the plasmid pTRC-BS, which is resistant to ampicillin.

In the examples of the present invention, wild-type P450BM3 (nucleotide sequence SEQ ID No. 1, amino acid sequence SEQ ID No. 2) was provided, and then the gene encoding the P450BM3 variant F87I/A82M/A330V was cloned into the pCDF-duet vector to create pCDF-FIAMAV. In the examples of the present invention, the T7 promoter (TAATACGACTCACTATAGGGGAA) of pCDF-FIAMAV was mutated (TAATACGACTCACTATCAAGGAA) to generate pCDF-FIAMAV-V1. pCDF-duet is resistant to streptomycin.

### Comparative Example 1: Single-strain system

A production strain was obtained by transforming the pMVA, pTRC-BS, and pCDF-FIAMAV or pCDF-FIAMAV-V1 plasmids into *E. coli* BL21 (DE3). The single strain was resistant to ampicillin, chloramphenicol, and streptomycin. Colonies of the production strain were inoculated into 5 mL of LB (Luria-Bertani) medium containing 100 µg/mL ampicillin, 36 µg/mL chloramphenicol, and 100 µg/mL streptomycin, and incubated at 37°C for 12 hours to obtain overnight seed cultures. The overnight seed cultures were inoculated into 25 mL of TB (Terrific Broth) medium supplemented with 100 µg/mL ampicillin, 36 µg/mL chloramphenicol, and 100 µg/mL streptomycin, and incubated at 37 °C and 200 rpm. Once the OD value at 600 nm reached 0.6, the cultures were induced with 0.1 mM IPTG and incubated at 30 °C. 2% w/v glucose was added as a carbon source, and 15% v/v decane was added as a second organic phase to extract terpenoids. Cultures were induced twice in 24-hour intervals with 0.1 mM IPTG and supplemented with 100 µg/mL ampicillin, 36 µg/mL chloramphenicol, 100 µg/mL streptomycin, 2% glucose, and 2% tryptone. The decane phase was collated by centrifugation after 72 hours and analyzed by GC.

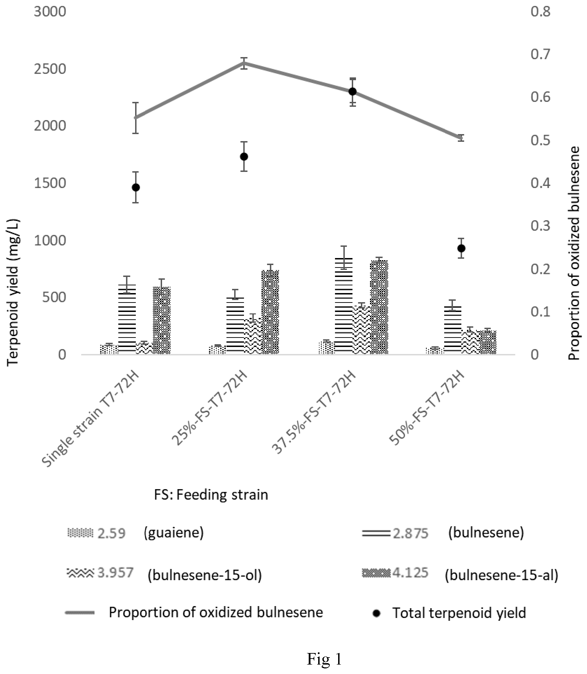

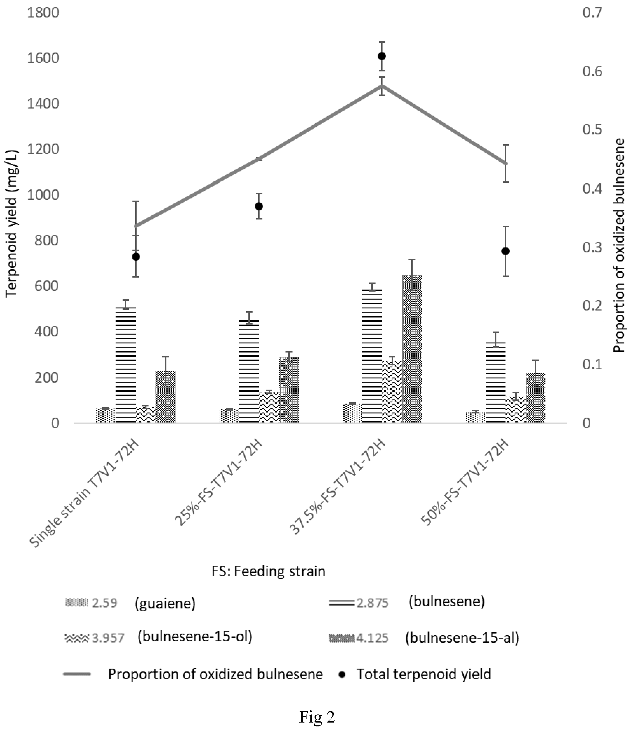

The single-strain system using pCDF-FIAMAV produced 1400 mg/L of total terpenoids and 53% oxidized bulnesene (bulnesene-15-ol and bulnesene-15-al) (Figure 1). The single-strain system using pCDF-FIAMAV-V1 produced 885 mg/L of total terpenoids and 36% oxidized bulnesene (Figure 2).

### Comparative Example 2: Co-cultivation system

A feeding strain was generated by transforming the pMevt, pUC-19, and pCDF-duet plasmids into *E. coli* BL21 (DE3). A production strain was obtained by transforming the pMBIS, pTRC-BS, and pCDF-FIAMAV or pCDF-FIAMAV-V1 plasmids into *E. coli* BL21 (DE3). Both the feeding and production strains wereresistant to ampicillin, chloramphenicol, and streptomycin. Colonies of the feeding and production strains were inoculated into separate 5 mL of LB (Luria-Bertani) medium supplemented with 100 µg/mL ampicillin, 36 µg/mL chloramphenicol, and 100 µg/mL streptomycin, and incubated at 37 °C for 12 hours to obtain overnight seed cultures. Different ratios (25%:75% to 50%:50%) of overnight cultures of the feeding and production strains were inoculated into 25 mL of TB (Terrific Broth) medium supplemented with 100 µg/mL ampicillin, 36 µg/mL chloramphenicol, and 100 µg/mL streptomycin, and incubated at 37 °C and 200 rpm. Once the OD value at 600 nm reached 0.6, the cultures were induced with 0.1 mM IPTG and incubated at 30 °C. 100 µg/mL ampicillin, 36 µg/mL chloramphenicol, 100 µg/mL streptomycin, and 2% w/v glucose as a carbon source were added, and 15% v/v decane was added as a second organic phase to extract terpenoids.

The co-cultivation system with pCDF-FIAMAV and 37.5% feeding strain produced 2234 mg/L of total terpenoids and 59% oxidized bulnesene (Figure 1). The co-cultivation system with pCDF-FIAMAV and 25% feeding strain produced 1667 mg/L of total terpenoids and 66% oxidized bulnesene (Figure 1). The co-cultivation system with pCDF-FIAMAV-V1 and 37.5% feeding strain produced 1600 mg/L of total terpenoids and 60% oxidized bulnesene (Figure 2).

### Example 1: Antibiotic control of 25% feeding strain and 75% production strain

A feeding strain was generated by transforming the pMevt and pUC-19, and plasmids into *E. coli* BL21 (DE3). Unlike in the comparative example, the feeding strain in this example does not contain the pCDF-duet plasmid, and was therefore sensitive to streptomycin. A production strain was generated by transforming the pMBIS, pTRC-BS, and pCDF-FIAMAV plasmids into *E. coli* BL21 (DE3). The feeding strain was sensitive to streptomycin, but due to the presence of pCDF-FIAMAV, the production strain was resistant thereto. Colonies of the feeding and production strains were inoculated into 5 mL of LB (Luria-Bertani) medium supplemented with 100 µg/mL ampicillin and 36 µg/mL chloramphenicol. Colonies of the production strain were inoculated into 5 mL of LB (Luria-Bertani) medium containing 100 µg/mL ampicillin, 36 µg/mL chloramphenicol, and 100 µg/mL streptomycin. The feeding and production strains were incubated at 37 °C for 12 hours to obtain overnight seed cultures. The feeding and production strains in a volume ratio of 25%:75% were inoculated into 25 mL of TB (Terrific Broth) medium supplemented with 100 µg/mL ampicillin and 36 µg/mL chloramphenicol, and incubated at 37 °C and 200 rpm, with the streptomycin concentration maintained at 10 µg/mL or 12.5 µg/mL. Once the OD value at 600 nm reached 0.6, the cultures were induced with 0.1 mM IPTG and incubated at 30 °C. 2% w/v glucose was added as a carbon source, and 15% v/v decane was added as a second organic phase to extract terpenoids. Cultures were induced twice in 24-hour intervals with 0.1 mM IPTG and supplemented with streptomycin (10 µg/mL or 12.5 µg/mL), 100 µg/mL ampicillin, 36 µg/mL chloramphenicol, 2% glucose, and 2% tryptone. The decane phase was collated by centrifugation after 72 hours and analyzed by GC.

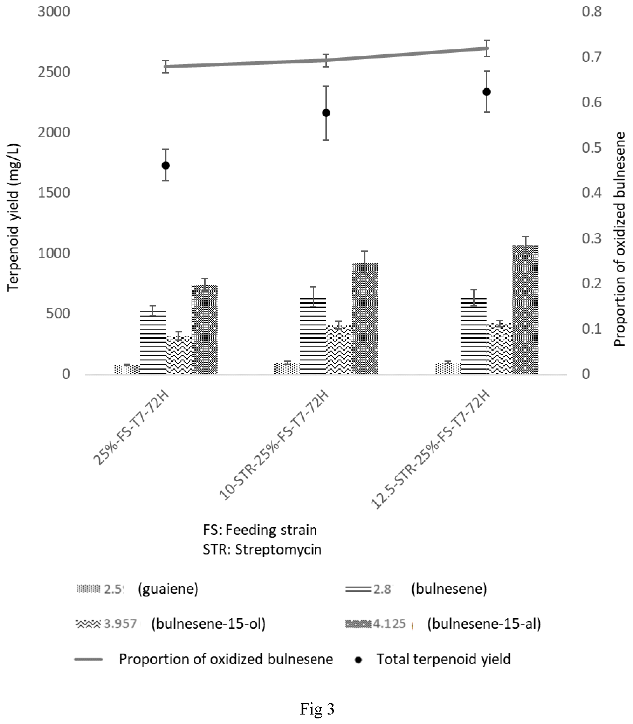

The antibiotic-controlled co-cultivation system with 12.5 µg/mL streptomycin produced 2224 mg/L of total terpenoids and 70% oxidized bulnesene (Figure 3). Compared with the co-cultivation system having the same inoculation ratio, the total terpenoids yield increased by 33% (Figure 3).

**Example 2: Antibiotic control of 37.5% feeding strain and 62.5% production strain** A feeding strain was generated by transforming the pMevt and pUC-19, and plasmids into *E. coli* BL21 (DE3). Unlike in the comparative example, the feeding strain in this example does not contain the pCDF-duet plasmid and was therefore sensitive to streptomycin. A production strain was generated by transforming the pMBIS, pTRC-BS, and pCDF-FIAMAV plasmids into *E. coli* BL21 (DE3). The feeding strain was sensitive to streptomycin, but due to the presence of pCDF-FIAMAV, the production strain was resistant thereto. Colonies of the feeding strain were inoculated into 5 mL of LB (Luria-Bertani) medium containing 100 µg/mL ampicillin and 36 µg/mL chloramphenicol. Colonies of the production strain were inoculated into 5 mL of LB (Luria-Bertani) medium containing 100 µg/mL ampicillin, 36 µg/mL chloramphenicol, and 100 µg/mL streptomycin. The feeding and production strains were incubated at 37 °C for 12 hours to obtain overnight seed cultures. The feeding and production strains in a volume ratio of 37.5%:62.5% were inoculated into 25 mL of TB medium and incubated at 37 °C and 200 rpm, with the streptomycin concentration maintained at 10 µg/mL to 15 µg/mL. Once the OD value at 600 nm reached 0.6, the cultures were induced with 0.1 mM IPTG and incubated at 30 °C. 2% w/v glucose was added as a carbon source, and 15% v/v decane was added as a second organic phase to extract terpenoids. Cultures were induced twice in 24-hour intervals with 0.1 mM IPTG and supplemented with 100 µg/mL ampicillin, 36 µg/mL chloramphenicol, streptomycin (10 µg/mL to 15 µg/mL), 2% glucose, and 2% tryptone. The decane phase was collated by centrifugation after 72 hours and analyzed by GC.

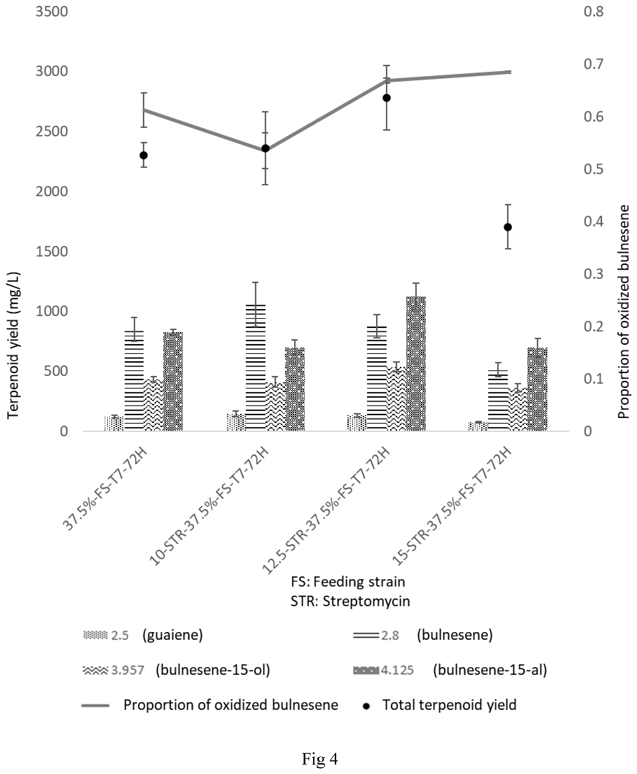

The antibiotic-controlled co-cultivation system using 12.5 µg/mL streptomycin produced 2673 mg/L of total terpenoids and 65% oxidized bulnesene (Figure 4). Compared with the co-cultivation system having the same inoculation ratio, the total terpenoids yield increased by 15% (Figure 4).

### Example 3: Antibiotic control of 50% feeding strain and 50% production strain

A feeding strain was generated by transforming the pMevt and pUC-19, and plasmids into *E. coli* BL21 (DE3). Unlike in the comparative example, the feeding strain in this example does not contain the pCDF-duet plasmid and was therefore sensitive to streptomycin. A production strain was generated by transforming the pMBIS, pTRC-BS, and pCDF-FIAMAV plasmids into *E. coli* BL21 (DE3). The feeding strain was sensitive to streptomycin, but due to the presence of pCDF-FIAMAV, the production strain was resistant thereto. Colonies of the feeding strain were inoculated into 5 mL of LB (Luria-Bertani) medium containing 100 µg/mL ampicillin and 36 µg/mL chloramphenicol. Colonies of the production strain were inoculated into 5 mL of LB (Luria-Bertani) medium containing 100 µg/mL ampicillin, 36 µg/mL chloramphenicol, and 100 µg/mL streptomycin. The feeding and production strains were incubated at 37 °C for 12 hours to obtain overnight seed cultures. The feeding and production strains in a volume ratio of 50%:50% were inoculated into 25 mL of TB medium supplemented with 100 µg/mL ampicillin and 36 µg/mL chloramphenicol, and incubated at 37 °C and 200 rpm. The streptomycin concentration was maintained at 10 µg/mL to 15 µg/mL. Once the OD value at 600 nm reached 0.6, the cultures were induced with 0.1 mM IPTG and incubated at 30 °C. 2% w/v glucose was added as a carbon source, and 15% v/v decane was added as a second organic phase to extract terpenoids. Cultures were induced twice in 24-hour intervals with 0.1 mM IPTG and supplemented with streptomycin (10 µg/mL to 15 µg/mL), 100 µg/mL ampicillin, 36 µg/mL chloramphenicol, 2% glucose, and 2% tryptone. The decane phase was collated by centrifugation after 72 hours and analyzed by GC.

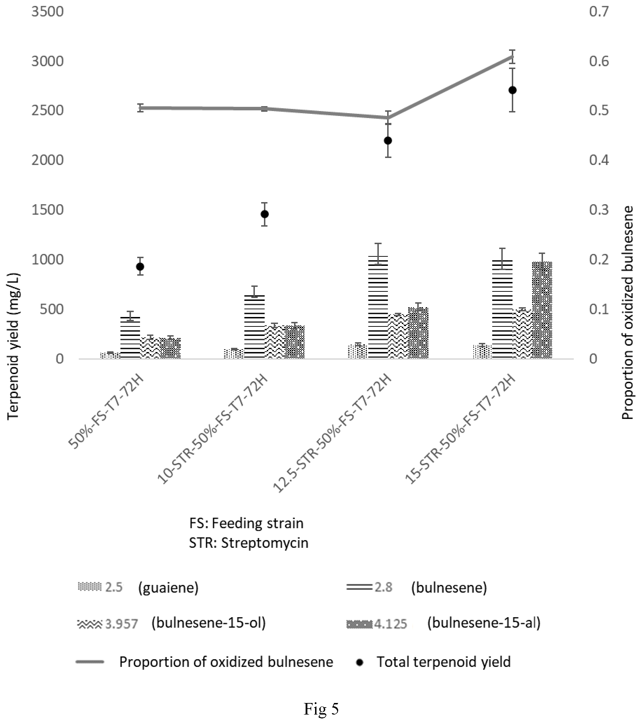

The antibiotic-controlled co-cultivation system using 15 µg/mL streptomycin produced 2627 mg/L of total terpenoids and 59% oxidized bulnesene (Figure 5). The total terpenoid yield was 2.5-fold higher than that of the co-cultivation system with the same inoculation ratio (Figure 5).

### Example 4: Increasing antibiotic concentration for 25% feeding strain and 75% production strain

A feeding strain was generated by transforming the pMevt and pUC-19 plasmids into *E. coli* BL21 (DE3). Unlike in the comparative example, the feeding strain in this example does not contain the pCDF-duet plasmid and was therefore sensitive to streptomycin. A production strain was generated by transforming the pMBIS, pTRC-BS, and pCDF-FIAMAV-V1 plasmids into *E. coli* BL21 (DE3). The pCDF-FIAMAV-V1 plasmid was created by mutating the T7 promoter to regulate FIAMAV expression. The feeding strain was sensitive to streptomycin, but due to the presence of pCDF-FIAMAV-V1, the production strain was resistant thereto. Colonies of the feeding strain were inoculated into 5 mL of LB (Luria-Bertani) medium containing 100 µg/mL ampicillin and 36 µg/mL chloramphenicol. Colonies of the production strain were inoculated into 5 mL of LB (Luria-Bertani) medium containing 100 µg/mL ampicillin, 36 µg/mL chloramphenicol, and 100 µg/mL streptomycin. The feeding and production strains were incubated at 37 °C for 12 hours to obtain overnight seed cultures. The feeding and production strains in a volume ratio of 25%:75% were inoculated into 25 mL of TB medium supplemented with 100 µg/mL ampicillin and 36 µg/mL chloramphenicol, and incubated at 37 °C and 200 rpm. The streptomycin concentration was maintained at 5 µg/mL or 10 µg/mL. Once the OD value at 600 nm reached 0.6, the cultures were induced with 0.1 mM IPTG and incubated at 30 °C. 2% w/v glucose was added as a carbon source, and 15% v/v decane was added as a second organic phase to extract terpenoids. Cultures were induced twice in 24-hour intervals with 0.1 mM IPTG and supplemented with 100 µg/mL ampicillin, 36 µg/mL chloramphenicol, 2% glucose, and 2% tryptone. After 24 hours, 10 µg/mL or 15 µg/mL streptomycin was added to the cultures. After 48 hours, 15 µg/mL to 25 µg/mL streptomycin was added to the cultures. The decane phase was collated by centrifugation after 72 hours and analyzed by GC.

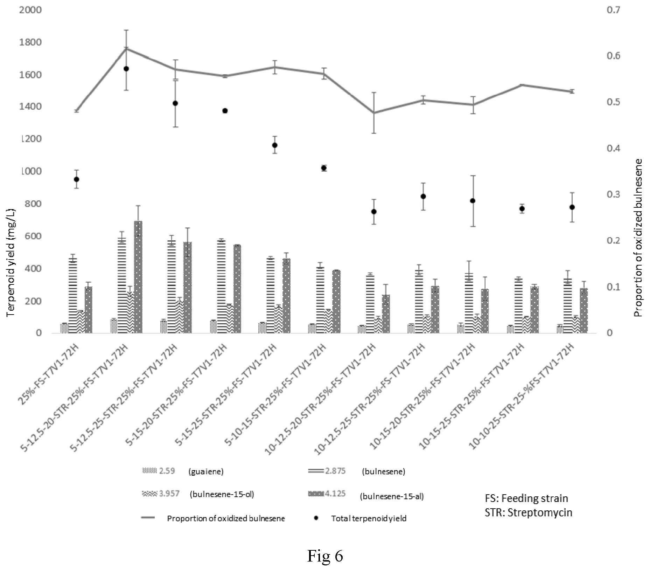

The antibiotic-controlled co-cultivation system with stepwise increases in streptomycin concentration (5 µg/mL at inoculation, 12.5 µg/mL at 24 hours, and 20 µg/mL at 48 hours) produced 1636 mg/L of total terpenoids and 61% oxidized bulnesene (Figure 6). Compared with the co-cultivation system having the same inoculation ratio, the total terpenoids yield increased by 70% (Figure 6).

While some exemplary embodiments of the present application have been illustrated and described, the present application is not limited to the disclosed embodiments. Rather, those of ordinary skill in the art will recognize that certain modifications and changes can be made to the described embodiments without departing from the spirit and scope of the application as described in the appended claims.

## Claims

1. A co-cultivation system for synthesizing a target compound, comprising at least one feeding strain and at least one production strain, wherein the feeding strain provides a precursor compound, and the production strain synthesizes the target compound using the precursor compound, the co-cultivation system further comprising an antibiotic, and wherein one of the production strain and the feeding strain is resistant to the antibiotic, and the other is not resistant to the antibiotic.

2. The co-cultivation system according to claim 1, wherein the target compound is a terpenoid; preferably, the precursor compound is mevalonic acid, the feeding strain is used to provide mevalonic acid, and the production strain is used to synthesize the terpenoid from the mevalonic acid.

3. The co-cultivation system according to claim 1, wherein the target compound includes at least one of a monoterpenoid, a sesquiterpenoid, and a diterpenoid.

4. The co-cultivation system according to claim 3, wherein the monoterpenoid includes at least one of linalool, geraniol, and 2,6-dimethyloctane; the sesquiterpenoid includes at least one of bisabolene, pentalenene, isohimachalene, bulnesene, valencene, β-patchoulene, aristolochene, guaiene, cedrene, oxidized bulnesene, and oxidation products of valencene; and the diterpenoid is taxadiene.

5. The co-cultivation system according to claim 1 or 2, wherein the antibiotic is an intracellular antibiotic, preferably streptomycin.

6. The co-cultivation system according to claim 1, wherein, based on the co-cultivation system, the concentration of the antibiotic is at least 5 µg/mL, and/or at most 20 µg/mL.

7. The co-cultivation system according to claim 1, wherein, based on the co-cultivation system, the antibiotic concentration is from 5 µg/mL to 20 µg/mL, preferably from 5 µg/mL to 12.5 µg/mL, particularly preferably from 5 µg/mL to 10 µg/mL.

8. The co-cultivation system according to claim 1, wherein an antibiotic resistance plasmid has been removed from the feeding strain.

9. A method for synthesizing a target compound, wherein the method synthesizes the target compound by means of the co-cultivation system according to any one of claims 1 to 8.

10. The method according to claim 9, wherein the concentration of the antibiotic is set such that the ratio of the feeding strain to the production strain corresponds to the concentration of the antibiotic.

11. The method according to claim 9, wherein, when the inoculation ratio of the feeding strain to the production strain exceeds 25%:75%, the concentration of the antibiotic may also be gradually increased.
